(19) Europäisches Patentamt
European Patent Office
Office européen des brevets

(11) Veröffentlichungsnummer: **0 623 593 A2**

# (12) EUROPÄISCHE PATENTANMELDUNG

(21) Anmeldenummer: **94107011.2**

(22) Anmeldetag: **04.05.94**

(51) Int. Cl.5: **C07C 303/32**, C11D 1/12, C11D 1/29

(30) Priorität: **05.05.93 DE 4314885**

(43) Veröffentlichungstag der Anmeldung: **09.11.94 Patentblatt 94/45**

(84) Benannte Vertragsstaaten: **AT BE CH DE ES FR GB IT LI NL SE**

(71) Anmelder: **SÜD-CHEMIE AG**
**Lenbachplatz 6**
**D-80333 München (DE)**
Anmelder: **DALLI-WERKE WÄSCHE- und KÖRPERPFLEGE GmbH & Co. KG**
**Zweifaller Strasse 120**
**D-52220 Stolberg (DE)**

(72) Erfinder: **Hirsch, Rüdiger, Dr.**
**Höhenkreuzweg 74,**
**D-52223 Solberg (DE)**
Erfinder: **Schmitz, Herbert**
**Bergkamp 3,**
**D-52393 Hürtgenwald (DE)**
Erfinder: **Schall, Noebert, Dr.**
**Am Strogenkanal**
**D-85465 Langenpreising (DE)**

(74) Vertreter: **Splanemann, Rainer et al**
**Patentanwälte Dipl.-Ing. R. Splanemann**
**Dr. B. Reitzner**
**Dipl.-Ing. K. Baronetzky**
**Tal 13**
**D-80331 München (DE)**

## (54) Verfahren zur Neutralisation der Säureform von anionischen Tensiden, danach erhaltene Agglomerate und Waschmittel.

(57) Beschrieben wird ein Verfahren zur Neutralisation von im wesentlichen alkylbenzolsulfonsäurefreien anionischen Tensiden mit einer basisch reagierenden Alkaliverbindung, das dadurch gekennzeichnet ist, daß man die Alkaliverbindung trocken mit einem pulverförmigen Trägermaterial vermischt, die hydrolyseempfindliche Säureform eines hydrolyseempfindlichen anionischen Tensids mit dieser Mischung unter Bildung eines Agglomerats ohne Freisetzung von Wasser umsetzt und das Tensid auf dem Agglomerat fixiert.

Das nach diesem Verfahren erhältliche Agglomerat kann in einem Waschmittel neben zusätzlichen üblichen, aber auch besonders feuchtigkeitsempfindlichen Waschmittelkomponenten eingesetzt werden.

EP 0 623 593 A2

Die Erfindung betrifft ein Verfahren zur Neutralisation der Säureform von anionischen Tensiden mit einer basisch reagierenden Alkaliverbindung, nach diesem Verfahren erhältliche Agglomerate sowie diese Agglomerate enthaltende Waschmittel.

Die Neutralisation von organischen Schwefelsäuren oder Sulfosäuren in wäßrigen Medien ist allgemein bekannt (vgl. z.B. DE-A-27 28 973, GB-A-819 183, GB-A-1 052 976, US-A-2 975 141, DE-A-4 017 463). Ferner ist bekannt, saure Alkylsulfate mit fester Soda umzusetzen, wobei trockene Natriumalkylsulfate pulverförmig anfallen (K. Lindner, Tenside, Textilhilfsmittel, Waschhilfsstoffe, Band 1, Wissenschaftliche Verlagsgesellschaft mbH, Stuttgart (1964), S. 646).

Aus der DE-B-853 443 ist ein Verfahren zur kontinuierlichen Neutralisation von Sulfosäuren und Schwefelsäureestern bekannt, wobei Sulfosäuren oder Schwefelsäureester in feiner Verteilung gleichzeitig mit fein gemahlenen trockenen Alkalicarbonaten in mindestens äquivalenter Menge derart zerstäubt werden, daß eine innige Vermischung der Reaktionsteilnehmer zustande kommt. Der Überschuß der Alkalicarbonate ist doch verhältnismäßig gering. Bei der Neutralisation wird Wasser frei; ferner können bis zu 15% Wasser zugesetzt werden.

Eine Weiterentwicklung dieses Verfahrens ist in der DE-B-868 907 beschrieben. Bei der Neutralisation wird ein Alkalicarbonatüberschuß von etwa 15% verwendet, und das Reaktionsprodukt wird nach einer zwischengeschalteten Homogenisierung durch Zusatz von Säure auf den gewünschten pH-Wert eingestellt. Das gebildete Neutralisationswasser wird durch Trocknung entfernt.

Aus der DE-A-2 203 552 ist ein Verfahren zum Neutralisieren eines synthetischen organischen, anionischen, in Säureform vorliegenden Tensids durch Zugabe zu einem Überschuß eines gepulverten Neutralisierungsmittels bekannt. Die Tenside liegen nicht in einer hydrolyseempfindlichen Säureform vor, und bei der Neutralisation wird Wasser freigesetzt, das als Dampf mit Hilfe eines strömenden Gases entfernt wird. Es wird während der Neutralisation kein pulverförmiges Trägermaterial verwendet. Das in Säureform vorliegende Tensid wird mit Wasser gewaschen, was bedeutet, daß es nicht hydrolyseempfindlich ist.

Aus der EP-A-0 050 897 ist ein stabiles, schnell dispergierbares Zeolith-Builder-Agglomerat bekannt, bei dessen Herstellung dem Zeolith etwa 1-4% eines nicht hydrolyseempfindlichen anionischen Tensids in der Säureform zugesetzt werden, um die Benetzbarkeit des Zeoliths zu verbessern. Das Zeolith-Builder-Agglomerat enthält ferner etwa 1-10% eines anorganischen Salzes, wie Natriumcarbonat. Bei der Neutralisation wird Wasser frei, das bei Verwendung eines hydrolyseempfindlichen anionischen Tensids schädlich wäre.

Aus der US-A-4 935 158 und der US-A-5 108 642 sind feste Tensid-Zusammensetzungen in Form eines Scheuerkissens bekannt, die als anionisches Tensid Alkylbenzolsulfonsäure enthalten, welche nicht hydrolyseempfindlich ist. Als Neutralisationsmittel wird beispielsweise Natriumcarbonat in einem geringen Überschuß sowie ein Füllstoff, wie Natriumsulfat, verwendet.

Anionische Tenside aus der Substanzgruppe der Fettalkoholsulfate bzw. Fettalkoholethersulfate haben in neuerer Zeit aufgrund günstiger ökologischer Bewertungen (Herstellung aus nachwachsenden Rohstoffen und vollständiger aerober und anaerober Abbau) gegenüber den bisher am häufigsten verwendeten Alkylbenzolsulfonaten ein starkes Interesse gefunden. Ihre Verwendbarkeit in Waschmitteln war jedoch wegen ihrer Hydrolyseempfindlichkeit sowie aus anderen, nachstehend noch näher erläuterten Gründen, begrenzt.

Bei diesen hydrolyseempfindlichen Tensiden handelt es sich z.B. um Alkylsulfate der Formel

$R_1$-(E)$_n$-$OSO_3$ M

worin $R_1$ eine Alkylgruppe mit 8 bis 22 Kohlenstoffatomen, E die Gruppe -($OCH_2$-$CH_2$)- , n = 0 bis 20 und M ein Alkalimetall wie Na, K oder Li bedeuten.

Hydrolyseempfindliche anionische Tenside sind ferner (vergl. WO 92/06170) α-sulfonierte Fettsäurealkylester mit der Formel

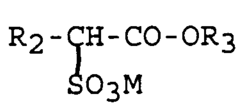

worin $R_2$ eine Alkylgruppe mit 7 bis 21 Kohlenstoffatomen, $R_3$ eine Alkylgruppe mit 1 bis 5 Kohlenstoffatomen und M eine Alkalimetall, wie Na, K oder Li bedeuten.

Die anionischen Tenside werden durch Neutralisation ihrer Säureform, im ersteren Fall beispielsweise der Substanzgruppe der Monoalkanolschwefelsäureester, hergestellt. Diese haben die Formel

$R_1(E)_n$-$OSO_2$-OH

worin $R_1$, E und n die vorstehend angegebene Bedeutung haben.

Die Säureform dieser Tenside ist nur begrenzt lagerstabil. Durch Hydrolyse entstehen bereits in Gegenwart von Spuren von Wasser der entsprechende Alkohol und Schwefelsäure.

Bei den α-sulfonierten Fettsäurealkylestern der Formel

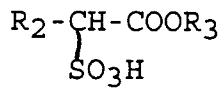

worin $R_2$ und $R_3$ die vorstehend angegebenen Bedeutungen haben, kann eine Esterspaltung und teilweise die Hydrolyse am α-Kohlenstoff unter Freisetzung von Schwefelsäure stattfinden.

Wird mit NaOH neutralisiert, so entsteht bei beiden Säureformen Wasser als Reaktionsprodukt, wodurch einerseits die Hydrolysegefahr erhöht, andererseits die Verfügbarkeit des Tensids in hochkonzentrierter, wasserfreier Form eingeschränkt wird.

Auch die Tenside in der Salzform unterliegen in Gegenwart von Wasser sowie bei Mißachtung einiger Vorsichtsmaßnahmen bei der Lagerung der Gefahr einer Hydrolyse. Die notwendigen Vorsichtsmaßnahmen ergeben sich dadurch, daß die Tenside bei erhöhter Temperatur gelagert werden müssen. Beim Unterschreiten der empfohlenen Temperatur würde das Tensid eine erhöhte Viskosität aufweisen bzw. fest werden. Der Gefahr der Hydrolyse des Tensids muß daher durch eine ständige Kontrolle des pH-Wertes begegnet werden, wobei ein Alkaliüberschuß eingehalten werden muß. Zur Vermeidung von lokal zu niedrigen pH-Werten muß das Tensid umgepumpt werden.

Allgemein formuliert, hängt die Konsistenz einer Paste des Tensids von folgenden Parametern ab: dem Aktivgehalt (Tensidgehalt), dem Elektrolytgehalt, dem Anteil des bei der Sulfatierung nicht umgesetzen Fettalkohols, der freien Alkalität, und der Temperatur. Diese Parameter müssen daher zur Gewährleistung der Handhabung beeinflußt und kontrolliert werden.

Die Gegenwart des über die Neutralisation eingebrachten Wassers verursacht bei Fettalkoholsulfaten darüberhinaus Viskositätsanomalien. So ist das Fettalkoholsulfonat in wasserfreier Form bei Raumtemperatur ölig bis fest. Mit zunehmendem Wassergehalt sinkt die Viskosität zunächst ab, um beim Überschreiten eines für das Tensid typischen Wassergehalts (abhängig von der Kettenlänge des Alkylrestes) wieder anzusteigen. Auf diese Weise ergeben sich zwei Bereiche niedriger Viskosität.

Zur Vermeidung von Viskositätsproblemen wurde deshalb bereits vorgeschlagen (WO 92/06 170-A) eine aus einer Cosulfonierung von Alkylbenzolen und Fettalkoholen entstandene Mischung der Säureformen von Alkylbenzolsufonaten und Fettalkoholsulfaten zusammen zu neutralisieren, wobei das Verhältnis zwischen Alkylbenzolsulfonaten und Fettalkoholsulfaten etwa 5-50:2-40 beträgt. Der Nachteil dieser Mischungen besteht jedoch darin, daß die darin enthaltenen Alkylbenzolsulfonate ökologisch bedenklich sind.

Fettalkoholsulfate sind weiterhin thermisch empfindlich. Daher sind Verfahren erwünscht, bei denen das Wasser schonend entfernt wird. Dies ist z.B. bei der Sprühtrocknung eines Waschpulvers, in welchem die Fettalkoholsulfate als Tensidkomponente eingesetzt werden, nicht der Fall.

Fettalkoholsulfate haben im Wasser insbesondere bei niedrigen Temperaturen eine begrenzte Löslichkeit. Dies ist insbesondere in Waschmittelkonzentraten ein Nachteil, bei denen auf eine schnelle Löslichkeit bzw. Dispergierbarkeit der einzelnen Komponenten größter Wert gelegt werden muß.

Nach den üblichen Verfahren zur Herstellung von Fettalkoholsulfaten (vergl. z.B. Stache, Tensidtaschenbuch, 7. Ausgabe, Verlag Hanser (1981), Seiten 142 bis 146) wird die Säureform des Fettalkoholsulfats mit überschüssiger wäßriger Natronlauge zu Fettalkoholsulfat neutralisiert, wobei das Neutralisationsgemisch größere Mengen Wasser enthält. Dadurch erhöht sich die Viskosität, so daß die Fettalkoholsulfat-Paste bei höheren Temperaturen gelagert und zum Waschmittelhersteller transportiert werden muß. Wird die Paste beim Waschmittelhersteller mit den übrigen Waschmittelkomponenten vermischt und durch Heißsprühtrocknung getrocknet, so besteht die Gefahr einer thermischen Zersetzung des Tensids.

Der Erfindung lag daher die Aufgabe zugrunde, ein neutralisiertes anionisches Tensid in einer leicht handhabbaren und hydrolysebeständigen wasserfreien bzw. wasserarmen Anwendungsform bereitzustellen. Ferner soll die Wasserlöslichkeit des Tensids in einem Waschmittel verbessert werden und schließlich soll eine Waschmittelkomponente mit hohem Schüttgewicht erhalten werden.

Diese Aufgabe wird bei einem Verfahren zur Neutralisation der Säureform von im wesentlichen alkylbenzolsulfonsäurefreien anionischen Tensiden mit einer basisch reagierenden Alkaliverbindung erfindungsgemäß dadurch gelöst, daß man die Alkaliverbindung trocken mit einem pulverförmigen Trägermaterial vermischt, die hydrolyseempfindliche Säureform eines hydrolyseempfindlichen anionischen Tensids mit dieser Mischung unter Bildung eines Agglomerats ohne Freisetzung von Wasser umsetzt und das Tensid auf dem Agglomerat fixiert.

Vorzugsweise setzt man ein wasserunlösliches, pulverförmiges Trägermaterial oder ein Trägermaterial-Gemisch mit mindestens einer wasserunlöslichen Komponente ein.

Unter dem Begriff "im wesentlichen alkylbenzolsulfonsäurefrei" versteht man, daß die Säureform des anionischen Tensids weniger als 5 Gew.-%, vorzugsweise weniger als 3 Gew.-%, insbesondere weniger als 1 Gew.-% Alkylbenzolsulfonsäure bzw. Alkylbenzolsulfonate enthält.

Nach einer Abwandlung dieses Verfahrens kann die basisch reagierende Alkaliverbindung auch als Trägermaterial fungieren. Diese Abwandlung ist dadurch gekennzeichnet, daß man das pulverförmige Trägermaterial ganz oder teilweise durch die basisch reagierende Alkaliverbindung ersetzt, wobei man die Alkaliverbindung mit einem molaren Überschuß von mehr als 5, insbesondere von mehr als 7, über die zur Neutralisation des anionischen Tensids erforderliche Menge einsetzt und daß man die erhaltene Mischung gegebenenfalls mit weiterem Trägermaterial vermischt.

Durch die Verwendung eines pulverförmigen Trägermaterials bzw. der überschüssigen Alkaliverbindung wird die Neutralisation der Säureform des Tensids in sehr schonender Weise durchgeführt; andererseits wird das entstandene, ebenfalls hydrolyseempfindliche Tensid auf einem wasserarmen bzw. wasserfreien anorganischen Trägermaterial bzw. auf der überschüssigen Alkaliverbindung aufgebracht und damit agglomeriert.

Durch das erhaltene Agglomerat wird das Tensid nunmehr in einer festen, gut schüttfähigen Anwendungsform bereitgestellt, die beim Lagern keiner Hydrolysegefahr mehr ausgesetzt sind und über Wasser keinen Effekt auf andere empfindliche Waschmittelkomponenten mehr ausüben können.

Das Agglomerat hat üblicherweise ein Schüttgewicht von 600 bis 1000 g/Liter, wodurch es zur Einarbeitung in Waschmittelkonzentrate bestens geeignet ist. Die durch die Viskosität der unbehandelten Tenside bedingten Handhabungsprobleme werden durch das Aufbringen auf das feste Trägermaterial vermieden. Eine nachträgliche Trocknung ist nicht erforderlich. Da auch die in üblicher Weise neutralisierte Form des anionischen Tensids nur über eine beschränkte Lagerfähigkeit verfügt, ist das erfindungsgemäße Verfahren insgesamt geeignet, die Lagerfähigkeit von anionischen, hydrolyseempfindlichen Tensiden zu verbessern und eine wasserfreie bzw. wasserarme Agglomeration für feuchtigkeitsempfindliche Komponenten eines Waschmittels durchzuführen.

Vorzugsweise setzt man die Säureform eines Tensids mit der Formel

$R_1$-(E)$_n$-$OSO_3H$ (I)

ein, worin $R_1$ eine Alkylgruppe mit 8 bis 22 Kohlenstoffatomen, E die Gruppe -($OCH_2$-$CH_2$)- und n = 0 bis 20 bedeuten.

Ferner kann man als Säureform eines Tensids einen $\alpha$-sulfonierten Fettsäurealkylester mit der Formel

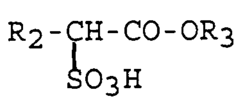
(II)

einsetzen, worin $R_2$ eine Alkylgruppe mit 7 bis 21 Kohlenstoffatomen und $R_3$ eine Alkylgruppe mit 1 bis 5 Kohlenstoffatomen bedeuten.

Zusätzlich zu der Säureform des anionischen Tensids können als Lösungsvermittler ein nichtionisches Tensid oder eine Fettsäure, die durch die basische Alkaliverbindung zu einer Seife neutralisiert wird, verwendet werden. Das nichtionische Tensid hat vorzugsweise die Formel

$R_4$-O($CH_2$-$CH_2$O)$_y$H (III)

worin $R_4$ eine Alkylgruppe mit 8 bis 22 Kohlenstoffatomen und y 1 bis 20 bedeuten.

Die als Lösungsvermittler verwendete Seife wird im Neutralisationsschritt über die Fettsäure dem System zugefügt.

Als basisch reagierende Alkaliverbindung verwendet man vorzugsweise ein Alkalicarbonat, wie Natriumcarbonat. Bei der Neutralisation bildet sich Natriumhydrogencarbonat, wobei kein Wasser freigesetzt wird.

Als Trägermaterial verwendet man vorzugsweise einen Smektit, Kaolin, ein schichtförmiges kristallines Alkalisilicat, ein amorphes festes Alkalisilicat, einen Zeolith, überschüssige trockene, basisch reagierende Alkaliverbindung, oder deren Gemische.

Beispiele für Smektite sind Montmorillonit, Beidellit und Hectorit. Vorzugsweise wird Montmorillonit verwendet, welcher ein quellfähiges, dioctaedrisches natürliches Schichtsilicat der allgemeinen Formel

$Na_{0,66}OH_4\,Si_8\,Al_{3,34}.Mg_{0,66})O_{20}$ darstellt.

Schichtförmige kristalline Alkalisilicate sind vorzugsweise solche aus der Gruppe der Phyllosilicate der Formel

$Me_2\,Si_xO_{2x+1}.H_2O$,

worin Me ein Alkalimetallion oder ein Proton, x eine Zahl > 7, insbesondere 7,5 bis 23, und y eine Zahl < 7x bedeuten. Typische kristalline Schichtsilicate aus dieser Gruppe sind in der DE-A-3 521 227 beschrieben. Sie sind unter der Bezeichnung SKS 6 (Firma Hoechst AG) im Handel.

Ein Beispiel für die festen amorphen Alkalisilicate, insbesondere für die Natriumsilicate, ist das Handelsprodukt "Brite-Sil H 20" der Firma Akzo N.V.

Als Zeolithe kann man beispielsweise den synthetischen Zeolith A, wie das Handelsprodukt "Wessalith P" (Firma Degussa AG) verwenden.

Bei der Abwandlung des erfindungsgemäßen Verfahrens, bei der das Trägermaterial ganz oder teilweise durch die trockene, basisch reagierende Alkaliverbindung ersetzt wird, wird als "Trägermaterial" vorzugsweise Natriumcarbonat in wasserfreier Form verwendet, das bei der Neutralisation ohne Freisetzung von Wasser in das Natriumhydrogencarbonat umgewandelt wird.

Die genannten Trägermaterialien können auch mit Polymeren, beispielsweise aus der Gruppe der Polyacrylate und Polymaleate, vermischt werden, wobei die Polymeren als Homopolymere und Copolymere mit einer Molmasse zwischen 2000 und 100.000 vorliegen können. Die Polymeren werden vorzugsweise zusammen mit den Trägermaterialien eingesetzt, die in einem Waschmittel als "Builder" (Wasserenthärter) wirken. Sie verhindern die Abscheidung von anorganischen Ausfällungen (Inkrustationen) auf dem Gewebe.

Zum Starten der Neutralisation kann man dem System eine kleine Menge Wasser oder einer Wasser enthaltenden Komponente, z.B. eine wäßrige Natriumsilicatlösung, zusetzen. Das Natriumsilicat dient auch zur Verbesserung der mechanischen Stabilität der Agglomerate.

Das erfindungsgemäße Verfahren wird vorzugsweise so durchgeführt, daß man das Trägermaterial und die basisch reagierende Alkaliverbindung vorlegt und die saure Form des anionischen Tensids unter intensivem Mischen bis zur Bildung des Agglomerats zusetzt. Die Agglomeratbildung kann ansatzweise oder kontinuierlich durchgeführt werden. Weiterhin kann das Agglomerat mit einem Rieselhilfsmittel, wie Kieselsäure, überzogen werden.

Gegenstand der Erfindung ist ferner ein Agglomerat, das nach dem vorstehend beschriebenen Verfahren erhältlich ist und das

(a) 0,5 bis 45 Gew.-% der neutralisierten Säureform des hydrolyseempfindlichen anionischen Tensids;

(b) eine basisch reagierende Alkaliverbindung in einem molaren Überschuß von mindestens 1,1 über die zur Neutralisation der Säureform des anionischen Tensids erforderlichen Menge;

(c) 20 bis 95 Gew.-% Trägermaterial und

(d) gegebenenfalls 0,5 bis 10 Gew.-% Rieselhilfsmittel enthält.

Vorzugsweise enthält das Agglomerat

(a) 2 bis 40 Gew.-%, vorzugsweise 5 bis 35 Gew.-%, der neutralisierten Säureform des anionischen Tensids;

(b) die basisch reagierende Alkaliverbindung in einem molaren Überschuß von 1,1 bis 3, vorzugsweise von 1,1 bis 2, über die zur Neutralisation der Säureform des anionischen Tensids erforderlichen Menge;

(c) 40 bis 80 Gew.-%, vorzugsweise 45 bis 75 Gew.-% Trägermaterial;

(d) gegebenenfalls 2 bis 7 Gew.-% Rieselhilfsmittel;

wobei für den Fall, daß das Trägermaterial ganz oder teilweise durch die basisch reagierende Alkaliverbindung ersetzt ist, die Menge der überschüssigen Alkaliverbindung der Menge des durch die Alkaliverbindung ersetzten Trägermaterials entspricht.

Gegenstand der Erfindung ist ferner ein Waschmittel, das das vorstehend beschriebene Agglomerat sowie zusätzliche Waschmittelkomponenten enthält.

Das erfindungsgemäße Verfahren kann einstufig oder zweistufig durchgeführt werden.

Die einfachere Variante ist das einstufige Verfahren. Zur Agglomeration nach dieser Verfahrensvariante kann sowohl ansatzweise als auch kontinuierlich gearbeitet werden. Arbeitet man ansatzweise, so kann man z.B. einen Eirich-Mischer verwenden, d.h. ein schräggestelltes, sich drehendes Gefäß, in dem das Mischgut zwangsweise einem sich hochtourig drehenden Wirbler zugeführt wird. Das Tensid wird in flüssiger Form eingespeist. Arbeitet man kontinuierlich, so kann man z.B. mit einem Schugi-Flexomix arbeiten, d.h. mit einem Fallrohr, bei dem das Pulver in eine Durchwirbelungszone fällt, in die das Tensid gesprüht wird und in der durch rotierende Werkzeuge eine Agglomerierung erfolgt.

Bei dem einstufigen Verfahren wird das Trägermaterial zunächst trocken mit der basisch reagierenden Alkaliverbindung vorgemischt, wobei die Alkaliverbindung gleichzeitig einen Teil des Trägermaterial bilden kann. Die Säureform des anionischen Tensids wird bei einer Wirblereinstellung von 5000 U/min dem Eirich-Mischer langsam zugegeben, bis das vorgelegte Pulver als Agglomerat vorliegt. Vor der Zugabe des anionischen Tensids kann eine Alkalisilicatlösung in einer Menge von 1 bis 4 Gew.-% zugegeben werden, wodurch die mechanische Stabilität des Agglomerats verbessert wird. Weiterhin kann zusätzlich zu der Säureform des anionischen Tensids ein Cotensid als Lösungsvermittler zugegeben werden, z.B. ein nichtionisches Fettalkoholethoxylat der Formel (III). Die Alkylkette des Fettalkoholethoxlats enthält 8 bis 22 Kohlenstoffatome, und die Anzahl der Ethoxygruppen beträgt 1 bis 20. Die Alkylgruppe kann geradkettig oder verzweigt sein. Als Lösungsvermittler kann auch die über den Neutralisationsschritt aus Fettsäure erhaltene Seife (mit 8 bis 22 Kohlenstoffatomen in der Kette) fungieren. Der Lösungsvermittler wird vorzugsweise in einer Menge von 1-20 Gew.-%, insbesondere von 5-10 Gew.-% der Säureform des anionischen Tensids verwendet.

Das erhaltene Agglomerat kann je nach der Menge des zudosierten Tensids und dem Charakter des Trägermaterials bereits in diesem Stadium gut schüttfähig, aber auch noch klebrig sein. Für den Fall, daß das Agglomerat klebrig ist, werden dem Mischer etwa 1 bis 10 Gew.-% eines Rieselhilfsmittels, wie Kieselsäure, zugesetzt; das Mischen wird über eine kurze Zeit, vorzugsweise über 10 bis 20 Sekunden, bei niedriger Umdrehungszahl, vorzugsweise 900 U/min, fortgesetzt, worauf ein farblich aufgehelltes, fließfähiges, nicht mehr klebriges Agglomerat erhalten wird.

Bei dem zweistufigen Verfahren wird die zweite Verfahrensvariante in Verbindung mit einem weiteren Verfahrensschritt angewendet. Hierbei wird die basisch reagierende Alkaliverbindung, vorzugsweise Soda, in einem hohen Überschuß über die Säureform des Tensids in einem Chargenmischer (z.B. Eirich-Mischer) oder in einem kontinuierlichen Mischer (z.B. Schugi-Flexomix) agglomeriert, wobei aber auf dieser Stufe noch Trägermaterial zugesetzt werden kann. Bei der Agglomeration wird die Säureform des Tensids zum Sulfat neutralisiert, gegebenenfalls in Kombination mit Fettsäure, die zu der entsprechenden Seife neutralisiert wird. Als Reaktionsprodukt entsteht Natriumhydrogencarbonat, ohne daß Neutralisationswasser freigesetzt wird. Das erhaltene Agglomerat stellt eine Basismischung dar, die in einem zweiten Verfahrensschritt mit einer Reihe von Zusätzen trocken gemischt werden kann.

Als Zusätze können die vorstehend genannten Trägermaterialien eingemischt werden, z.B. das schichtförmige kristalline Natriumsilicat, das potentiell von der Säureform des anionischen Tensids angegriffen werden kann. Weiterhin können die natürlichen Schichtsilicate, wie Smektite (Bentonit, Hectorit) oder Kaolin, zugesetzt werden, die ebenfalls potentiell von Säuren angegriffen werden, wenn auch in einem geringeren Ausmaß als das schichtförmige kristalline Natriumsilicat.

Bei diesem zweiten Verfahrensschritt können weiterhin geeignete Polymere, wie Acrylat- und Maleat-Homo- und Copolymere mit einer Molmasse zwischen 2000 und 100.000, nichtionische Tenside auf der Basis von Alkoholethoxylaten; Zeolith A, Kombinationen von Zeolith A mit Polymeren, amorphe feste Natriumsilicate und deren Mischungen mit Polymeren, Mischungen aus amorphen festen Natriumsilicaten und Natriumcitrat, auch mit Polymeren, zugesetzt werden. Das Natriumcitrat wirkt als Wasserenthärter.

Das Produkt aus dem einstufigen bzw. aus dem zweistufigen Verfahren ist ein Agglomerat, das als sogenanntes "Waschmittel-Compound" eingesetzt werden kann und das vorzugsweise 10 bis 35 Gew.-% der neutralisierten Säureform des anionischen Tensids, 5 bis 25 Gew.-% wasserfreie Soda (bzw. bis zu 80 Gew.-% Soda, wenn diese das Trägermaterial teilweise oder ganz ersetzt), 5 bis 15 Gew.-% Natriumhydrogencarbonat, 1 bis 5 Gew.-% Cotensid, 0,5 bis 10 Gew.-% Polymer und 45 bis 75 Gew.-% Trägermaterial enthält. Das Compound liegt als Agglomerat mit einem Schüttgewicht von 600 bis 1000 g/Liter vor. Der Wassergehalt des Compounds beträgt 0,1 bis 5 Gew.-%. Das erhaltene Compound eignet sich als Baustein für eine Waschmittelformulierung. Dieser Baustein erfüllt die Funktion der Tensid-Komponente und teilweise auch der Builder-Komponente und verleiht dem Waschmittel die notwendige Alkalität. Durch den sehr niedrigen Wassergehalt und das hohe Schüttgewicht ist die Kompatibilität für Kompaktwaschmittel gewährleistet. Darüber hinaus besteht wegen des niedrigen Wassergehalts Kompatibilität mit wasserempfindlichen Waschmittelinhaltsstoffen, z.B. Percarbonaten.

Die Erfindung ist durch die nachstehenden Beispiele in nicht einschränkender Weise erläutert.

Beispiel 1

In einen Eirich-Labormischer werden 515 g wasserfreie Soda und 1485 g pulverförmiges kristallines Natriumsilicat (Handelsprodukt SKS-6) trocken mit einer Umdrehungsgeschwindigkeit von 5000 U/min über einen Zeitraum von 20 sec miteinander vermischt. Dann werden 125 g einer 45 %igen Natrium-Wasserglaslösung über einen Zeitraum von 5 bis 10 sec zugemischt. In das Gemisch werden über einen Zeitraum von 3 min 680 g der Säureform eines anionischen Tensids gemäß Formel I, worin $R_1$ eine Alkylgruppe mit 16 bis 18 Kohlenstoffatomen darstellt, zusammen mit 90 g eines Cotensids aus der Gruppe der Fettalkoholethoxylate gemäß Formel III, worin $R_4$ eine Alkylgruppe mit 14 bis 15 Kohlenstoffatomen darstellt und $y = 4$ bedeutet, und 180 g eines Homopolymers der Maleinsäure mit einer Molmasse von etwa 4500 in Form einer 40 %igen wäßrigen Lösung eingearbeitet. Nach etwa einer halben Minute hat sich ein fließfähiges Agglomerat mit einer Schüttdichte von 740 g/l gebildet.

Beispiel 2

Die Arbeitsweise von Beispiel 1 wird mit der Abweichung wiederholt, daß statt des kristallinen Natriumsilicats 1485 g einer Zeolith A-Copolymer- Matrix, die in einem Sprühprozeß hergestellt wurde, verwendet wird. Als Copolymer wurde ein Acryl-Maleinsäure-Copolymer (Handelsbezeichnung : Socolan CP 5, Firma BASF) verwendet. Dieses Produkt wird mit 125 g einer 45 %igen Natrium-Wasserglaslösung, 90 g des nichtionischen Tensids von Beispiel 1 und 470 g der Säureform des anionischen Tensids von Beispiel 1 verarbeitet. Es wird hier kein flüssiges Polymer eingearbeitet, da dieses bereits im Zeolith A-Copolymer-Sprühanteil vorhanden ist. Ansonsten erfolgt die Verarbeitung wie nach Beispiel 1. Es resultiert ein Produkt mit einer Schüttdichte von 760g/l.

Beispiel 3

Nach der Arbeitsweise von Beispiel 1 werden 515 g wasserfreie Soda, 1485 g amorphes Alkalisilicat (Britesil H20), 600 g Na-Citrat, 125 g einer 45 %igen Natrium-Wasserglaslösung, 90 g des Cotensids und 500 g der Säureform des anionischen Tensids von Beispiel 1, sowie 270 g einer 40 %igen wäßrigen Lösung des Copolymers von Beispiel 2 verarbeitet. Es resultiert ein Agglomerat mit einer Schüttdichte von 760 g/l.

Beispiel 4

Nach der Arbeitsweise von Beispiel 1 werden 600 g wasserfreie Soda, 800 g Britesil H20, 600 g Na-Citrat, 125 g einer 45 %igen Natrium-Wasserglaslösung, 120 g des Cotensids und 440 g der Säureform des anionisches Tensids von Beispiel 1 sowie 195 g einer 40 %igen wäßrigen Lösung des Copolymers von Beispiel 2 verarbeitet. Es resultiert ein Agglomerat mit einer Schüttdichte von 700 g/l.

Beispiel 5

Nach der Arbeitsweise von Beispiel 1 werden 620 g Bentonit, 1380 g wasserfreie Soda, 90 g des Cotensids von Beispiel 1, 125 g einer 45 %igen Natrium-Wasserglaslösung und 920 g der Säureform des anionisches Tensids von Beispiel 1 zur Reaktion gebracht. Es resultiert ein Agglomerat mit einer Schüttdichte von 800 g/l.

Beispiel 6

Nach der Arbeitsweise von Beispiel 1 werden 590 g Kaolin, 1410 g wasserfreie Soda, 90 g des Cotensids von Beispiel 1, 125 g einer 45 %igen Natrium-Wasserglaslösung und 545 g der Säureform des anionischen Tensids von Beispiel 1 zur Reaktion gebracht. Es resultiert ein Agglomerat mit einer Schüttdichte von 750 g/l.

Beispiel 7

Nach der Arbeitsweise von Beispiel 1 werden 2000 g wasserfreie Soda mit 90 g des Cotensids von Beispiel 1 und 125 g einer 45 %igen Natrium-Wasserglaslösung mit 650 g der Säureform des anionischen Tensid von Beispiel 1 zur Reaktion gebracht. Es resultiert ein Agglomerat mit einer Schüttdichte von 650 g/l.

Alle Agglomerate nach den Beispielen 1 - 7 werden zusätzlich bei verminderter Umdrehungszahl (500 U/min im Eirich-Mischer) mit 80 g Kieselsäure vermischt und abgepudert. Die so behandelten Agglomerate werden nach etwa 10 sec aus dem Mischer entnommen. Die Produkte sind farblich heller und leicht rieselfähig bei praktisch unveränderter Schüttdichte.

Beispiel 8

In einem Eirich-Mischer werden 470 g wasserfreie Soda und 1530 g pulverförmiges kristallines Natriumsilicat (Handelsprodukt SKS-6) trocken mit einer Umdrehungsgeschwindigkeit von 5000 U/min 20 sec miteinander vermischt.

Anschließend werden 125 g einer 45 %igen Natrium-Wasserglasslösung über einen Zeitraum von 5 - 10 sec zugesetzt. In das Gemisch werden über 3 - 5 min 480 g der Säureform des anionischen Tensids der Formel I, worin $R_1$ eine Alkylgruppe mit 16 bis 18 Kohlenstoffatomen und n = 2 bedeuten, zusammen mit 50 g eines Cotensids aus der Gruppe der Fettalkoholethoxylate der Formel III, worin $R_4$ eine Alkylgruppe mit 14 bis 15 Kohlenstoffatomen und y = 4 bedeuten, und 150 g einer 40 %igen wäßrigen Lösung des Copolymers von Beispiel 2 eingearbeitet.

Nach etwa einer halben Minute hat sich ein fließfähiges Agglomerat mit einer Schüttdichte von 720 g/l gebildet, welches bei verminderter Umdrehungszahl (500 U/min) mit 80 g Kieselsäure vermischt und abgepudert wird.

Das so behandelte Agglomerat wird nach etwa 10 sec dem Mischer entnommen und kann anschließend in einem Laborfließbett (z.B. der Firma Petsch) auf 1-2 % Restfeuchte getrocknet werden.

Der so erhaltene Tensid-Builder-Baustein ist weiß, gut rieselfähig und besitzt eine Schüttdichte von 700 g/l.

Beispiel 9

Ein Gemisch aus wasserfreier Soda und pulverförmigem kristallinem Natriumsilicat (Handelsbezeichnung SKS-6) im Gewichtsverhältnis 1:1 wird mit einem Mengenstrom von 8,0 kg/min in einem kontinuierlichen Schugi-Flexomix-Mischer mit 0,26 kg/min einer 45 %igen Natrium-Wasserglaslösung vermischt. Das Pulvergemisch wird über eine Bandwaage zudosiert. Über ein zweites Düsensystem werden 3,0 kg/min eines Gemischs aus der Säureform des Tensids von Beispiel 1, des Cotensids der Formel III ($R_4$ = C14/15; y = 4) und der 40 %igen wäßrigen Copolymerlösung nach Beispiel 2 im Verhältnis 110:2:3 zudosiert. Das entstehende Feuchtagglomerat ist fließbettgeeignet und wird in einem nachgeschalteten Fließbett auf 1-2 % Restfeuchte getrocknet und auf 25 °C gekühlt.

Je nach Farbaspekt des Feuchtagglomerates kann dieses vor der Fließbettbehandlung noch mit Kieselsäure in einer Menge von etwa 38 g/kg vermischt und abgepudert werden.

Die Zusammensetzung des Compound nach dem Fließbett ist nachstehend angegeben.

| | | | |
|---|---|---|---|
| 4,00 | Gew.-Teile | SKS-6 | 36,5 % |
| 3,33 | " | $Na_2CO_3$ | 30,3 % |
| 0,53 | " | $NaHCO_3$ | 4,8 % |
| 0,12 | " | Na-Silicat | 1,1 % |
| 2,14 | " | Tensid Formel I (Na-Form) | 19,5 % |
| 0,40 | " | Cotensid Formel III | 3,6 % |
| 0,24 | " | Copolymer | 2,2 % |
| 0,22 | " | $H_2O$ | 2,0 % |
| 10,98 | Gew.-Teile | | 100 % |

Beispiel 10

Die Arbeitsweise von Beispiel 1 wird mit der Abweichung wiederholt, daß als Säureform eines anionischen Tensids der $\alpha$-Sulfotalgfettsäuremethylester (Formel II-$R_2$ = 16-18 C-Atome; $R_3$ = $CH_3$) verwendet wird. Man erhält ein Agglomerat mit einer Schüttdichte von 760 g/l.

**Patentansprüche**

1. Verfahren zur Neutralisation der Säureform von im wesentlichen alkylbenzolsulfonsäurefreien anionischen Tensiden mit einer basisch reagierenden Alkaliverbindung, dadurch gekennzeichnet, daß man die Alkaliverbindung trocken mit einem pulverförmigen Trägermaterial vermischt, die hydrolyseempfindliche Säureform eines hydrolyseempfindlichen anionischen Tensids mit dieser Mischung unter Bildung eines Agglomerats ohne Freisetzung von Wasser umsetzt und das Tensid auf dem Agglomerat fixiert.

2. Verfahren nach Anspruch 1, dadurch gekennzeichnet, daß man ein wasserunlösliches pulverförmiges Trägermaterial oder ein Trägermaterial in Form eines Gemisches mit mindestens einer wasserunlöslichen Komponente einsetzt.

3. Abwandlung des Verfahrens nach Anspruch 1 oder 2, dadurch gekennzeichnet, daß man das pulverförmige Trägermaterial ganz oder teilweise durch die basisch reagierende Alkaliverbindung ersetzt, wobei man die Alkaliverbindung in einem molaren Überschuß von mehr als 5, insbesondere von mehr als 7, über die zur Neutralisation des anionischen Tensids erforderliche Menge einsetzt, und daß man die erhaltene Mischung gegebenenfalls mit weiterem Trägermaterial vermischt.

4. Verfahren nach einem der Ansprüche 1 bis 3, dadurch gekennzeichnet, daß man die Säureform eines Tensids mit der Formel

   $R_1$-$(E)_n$-$OSO_3H$ (I)

   einsetzt, worin $R_1$ eine Alkylgruppe mit 8 bis 22 Kohlenstoffatomen, E die Gruppe-($OCH_2$-$CH_2$) und n = 0-20 bedeuten.

5. Verfahren nach einem der Ansprüche 1 bis 4, dadurch gekennzeichnet, daß man als Säureform des Tensids einen $\alpha$-sulfonierten Fettsäurealkylester mit der Formel

$$R_2\text{-}\underset{\displaystyle SO_3H}{CH}\text{-}CO\text{-}OR_3 \qquad (II)$$

einsetzt, worin $R_2$ eine Alkylgruppe mit 7 bis 21 Kohlenstoffatomen und $R_3$ eine Alkylgruppe mit 1 bis 5 Kohlenstoffatomen bedeuten.

**6.** Verfahren nach einem der Ansprüche 1 bis 5, dadurch gekennzeichnet, daß man zusätzlich zu der Säureform des anionischen Tensids als Lösungsvermittler ein nichtionisches Tensid oder eine Fettsäure, die durch die basische Alkaliverbindung zu einer Seife neutralisiert wird, verwendet.

**7.** Verfahren nach einem der Ansprüche 1 bis 6, dadurch gekennzeichnet, daß man ein nichtionisches Tensid der Formel

$R_4\text{-}O(CH_2\text{-}CH_2O)_yH$ (III)

einsetzt, worin $R_4$ eine Alkylgruppe mit 8 bis 22 Kohlenstoffatomen und y 1 bis 20 bedeuten.

**8.** Verfahren nach einem der Ansprüche 1 bis 7, dadurch gekennzeichnet, daß man als basisch reagierende Alkaliverbindung ein Alkalicarbonat einsetzt.

**9.** Verfahren nach einem der Ansprüche 1 bis 8, dadurch gekennzeichnet, daß man als Trägermaterial einen Smektit, Kaolin, ein schichtförmiges kristallines Alkalisilicat, ein amorphes festes Alkalisilicat, einen Zeolith, überschüssige trockene, basisch reagierende Alkaliverbindung oder deren Gemische einsetzt.

**10.** Verfahren nach einem der Ansprüche 1 bis 9, dadurch gekennzeichnet, daß man das Trägermaterial und die basisch reagierende Alkaliverbindung vorlegt und die saure Form des anionischen Tensids unter intensivem Mischen bis zur Bildung des Agglomerats zusetzt.

**11.** Verfahren nach einem der Ansprüche 1 bis 10, dadurch gekennzeichnet, daß man die Agglomeratbildung ansatzweise oder kontinuierlich durchführt.

**12.** Verfahren nach einem der Ansprüche 1 bis 11, dadurch gekennzeichnet, daß man das Agglomerat mit einem Rieselhilfsmittel, wie Kieselsäure, überzieht.

**13.** Agglomerat, das nach dem Verfahren nach einem der Ansprüche 1 bis 12 erhältlich ist und das
(a) 0,5 bis 45 Gew.-% der neutralisierten Säureform des hydrolyseempfindlichen anionischen Tensids;
(b) eine basisch reagierende Alkaliverbindung in einem molaren Überschuß von mindestens 1,1 über die zur Neutralisation der Säureform des anionischen Tensids erforderlichen Menge;
(c) 20 bis 95 Gew.-% Trägermaterial und
(d) gegebenenfalls 0,5 bis 10 Gew.-% Rieselhilfsmittel enthält.

**14.** Agglomerat nach Anspruch 13, dadurch gekennzeichnet, daß es
(a) 2 bis 40 Gew.-%, vorzugsweise 5 bis 35 Gew.-%, der neutralisierten Säureform des anionischen Tensids;
(b) die basisch reagierende Alkaliverbindung in einem molaren Überschuß von 1,1 bis 3, vorzugsweise von 1,1 bis 2, über die zur Neutralisation der Säureform des anionischen Tensids erforderlichen Menge;
(c) 40 bis 80 Gew.-%, vorzugsweise 45 bis 75 Gew.-% Trägermaterial;
(d) gegebenenfalls 2 bis 7 Gew.-% Rieselhilfsmittel enthält;
wobei für den Fall, daß das Trägermaterial ganz oder teilweise durch die basisch reagierende Alkaliverbindung ersetzt ist, die Menge der überschüssigen Alkaliverbindung der Menge des durch die Alkaliverbindung ersetzten Trägermaterials entspricht.

**15.** Waschmittel, enthaltend ein Agglomerat nach einem der Ansprüche 13 oder 14 sowie zusätzliche übliche Waschmittelkomponenten.